## Europäisches Patentamt
## European Patent Office
## Office européen des brevets

(11) Publication number: **0 130 441**
**B1**

(12) **EUROPEAN PATENT SPECIFICATION**

(45) Date of publication of patent specification: **27.04.88**

(51) Int. Cl.⁴: **G 01 L 7/08**

(21) Application number: **84106865.3**

(22) Date of filing: **15.06.84**

(54) **Pressure measuring system.**

(30) Priority: **30.06.83 SE 8303740**

(43) Date of publication of application:
**09.01.85 Bulletin 85/02**

(45) Publication of the grant of the patent:
**27.04.88 Bulletin 88/17**

(84) Designated Contracting States:
**BE CH DE FR GB IT LI LU NL**

(56) References cited:
**US-A-3 501 959**
**US-A-4 202 081**

(73) Proprietor: **Gambro Lundia AB**
**Box 10101**
**S-220 10 Lund (SE)**

(72) Inventor: **Claren, Jan Seivert**
**Protokollgränden 38**
**S-222 47 Lund (SE)**
Inventor: **Jeppsson, Jan-Bertil**
**Västkustvägen 86**
**S-234 00 Lomma (SE)**
Inventor: **Staehr, Peter Robert**
**Hästad 27:1**
**S-225 90 Lund (SE)**

(74) Representative: **Boberg, Nils Gunnar Erik**
**Gambro AB Patent Department Box 10101**
**S-220 10 Lund (SE)**

Courier Press, Leamington Spa, England.

## Description

### Technical field

The present invention relates to a pressure measuring system comprising a pressure-sensitive element and a container provided with at least one inlet, the container being intended to communicate through this inlet with the medium whose pressure is to be measured.

The system in accordance with the invention is intended in particular to be used in connection with extracorporeal treatment for measuring the pressure either in the blood or in some other fluid included in the treatment. The system can be used for example in connection with haemodialysis, PD haemofiltration or plasmafers.

### Background art

Normally the medium whose pressure is to be measured is connected up directly or via a filter to a pressure gauge. With such an arrangement positive as well as negative pressure can be measured. Certain coupling problems arise, however, if the medium containing parts such as e.g. a flexible blood tube, is intended to be used only once whilst the pressure gauge is intended to be used several times.

To prevent such coupling problems it is feasible to let the pressure be transferred mechanically from an elastic pressure pad to a pressure gauge, e.g. in the manner as described in the Swedish patent 403 004 or the corresponding US patent 4 194 974. Such systems, however, are less suitable for the measurement of negative pressures. Frequently they are only used, moreover, for checking that a maximum negative and/or positive pressure are not exceeded.

Another generally known method for measuring a negative pressure consists in comparing the negative pressure with a reference pressure so as to arrive at a positive pressure difference which can be measured by any suitable method. Such a procedure can easily become relatively complicated, as the absolute pressure is not measured directly.

### Disclosure of invention

As the result of the present invention a system is provided instead by means of which it is possible in a simple manner to measure positive as well as negative pressures by means of a very simple coupling between the part which contains the medium whose pressure is to be measured and a pressure-sensitive element, e.g. a wire strain pick-up or a piezoelectric element.

The pressure measuring system in accordance with the invention thus comprises the said pressure-sensitive element and a container provided with at least one inlet, the container being intended to communicate through this inlet with the medium whose pressure is to be measured.

The system in accordance with the invention is characterised in that a pressure-transmitting wall part of the container is adapted so that with the help of a negative pressure it is fastened on by suction to the pressure-sensitive element for measuring substantially the absolute pressure in the container.

This pressure-transmitting wall part of the container thus has to be elastic so that the forces arising in the same do not considerably affect the measurement of the absolute pressure in the container.

Preferably the pressure-sensitive element is sunk into a cavity into which at least a part of the said container can be inserted for contact between the pressure-sensitive element and the pressure-transmitting wall part. In such a construction the cavity can easily be provided with sealing elements which are adapted so as to separate in a sealing manner the pressure-sensitive element and the pressure-transmitting wall part from the outside atmosphere when the container is in inserted position in the cavity. In this manner an effective fastening on by suction can be obtained even at relatively low negative pressure.

The container is provided appropriately with an outlet so that the medium whose pressure is to be measured can flow through. In this way the container can readily be included as a natural component in the flexible tube system which is normally used in connection, for example, with haemodialysis and which is fixed to the front of the monitor which is intended to control the treatment. The pressure-sensitive element included in the present system is suitably adapted so that it is sunk into this monitor.

If the part of the container which is insertable into the cavity is designed in substantially the same shape as the cavity, the sealing of the pressure-transmitting surfaces in relation to the surrounding atmosphere is facilitated.

Should the container contain blood it is important that it has the smallest possible volume. This can be achieved in that it is made double-walled, so that the space containing the medium is considerably smaller than the actual cavity into which the container is intended to be inserted.

A simple design is obtained if the cavity as well as the part of the container insertable therein are of substantially cylindrical shape and are sealed against each other by means of one or more annular seals, e.g. O-rings.

The system in accordance with the invention can comprise, moreover a thin and elastic protection sleeve of substantially the same shape as the cavity arranged between the cavity and the part of the container insertable therein. Thanks to this protective sleeve the pressure-sensitive body is protected in respect of leakage. Should the protective sleeve become contaminated, it can thus easily be substituted and will not affect the measurement.

A particularly simple construction is obtained if this protective sleeve is provided with the said sealing elements for bringing about the seal between the cavity and the part of the container insertable therein. These sealing elements may be in the shape, for example, of one or more sealing rings moulded integrally with the sleeve.

The inner part of the protective sleeve situated in inserted position closest to the pressure-sensitive element is preferably perforated so as to allow a tight fastening on by suction of the pressure-transmitting wall part of the container to the pressure-sensitive element essentially independently of the perforated part of the protective sleeve situated in between.

Brief description of drawings

The invention will be described in more detail in the following with reference to the enclosed drawings which show different parts which may be included in a preferred system in accordance with the invention.

Fig. 1 shows a set of parts in assembled condition which together form a relatively complete system.

Fig. 2 and 3 show two views perpendicular to one another of a container forming part of the system in accordance with the invention.

Fig. 4 and 5 show sections along the lines IV—IV and V—V respectively in Fig. 2.

Fig. 6 shows, seen from the top, half of a protective sleeve which may form part of the system in accordance with the invention.

Fig. 7 and 8 finally show in section two alternative embodiments of this protective sleeve.

Best mode of carrying out the invention

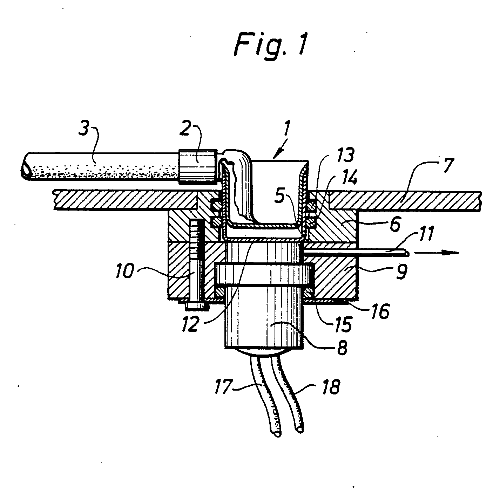

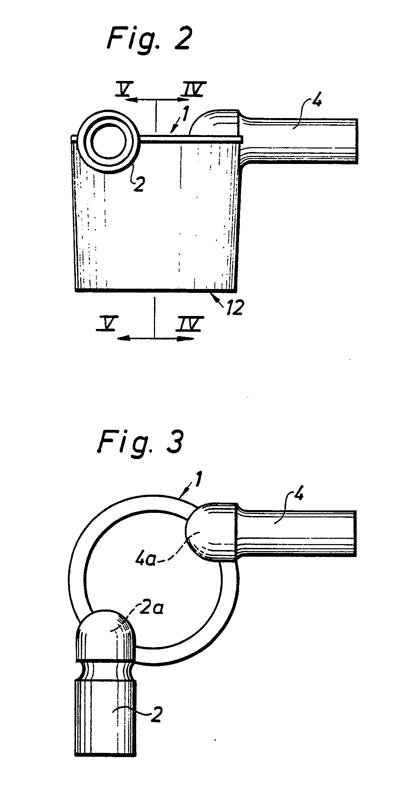

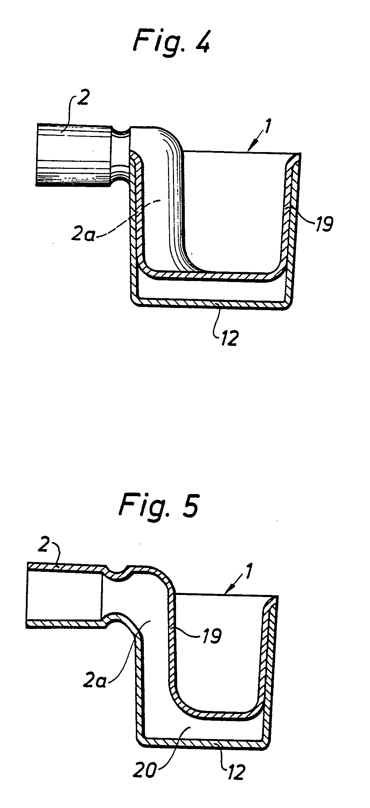

In Fig. 1 is thus shown a container 1 with an inlet 2 which via a flexible tube communicates with the medium whose pressure is to be measured. As will be described in more detail in connection with Fig. 2—4, the container 1 also comprises an outlet 4. It is intended thus that the medium whose pressure is to be measured should flow through the container.

In operating condition the container 1 is sunk into a cavity 5 in a part of the machine 6 which is fixed into a front plate 7, e.g. the front of a monitor for the control of haemodialysis or haemofiltration. In the machine part 6 a pressure-sensitive element 8 is fixed with the help of a further machine component 9. This component may be screwed on, for example, by means of a bolt 10. The pressure-sensitive element may be constituted, for example of a piezoelectric pressure pick-up. Alternatively it may comprise a wire strain pick-up or some other suitable pressure measuring element. By means of a duct 11 the cavity 5 may be put into communication with a source of vacuum (not shown). In that manner a pressure-transmitting wall part 12 of the container 1 is fastened by suction onto the pressure-sensitive element 8. The pressure-transmitting wall part 12 and the pressure-monitoring surface of the pressure-sensitive element 8 are sealed against the surrounding atmosphere with the help of annular seals 13, 14 and 15. The last-mentioned seal 15 is thus situated inside a lockwasher 16. Finally an electric connection to the pressure-sensitive element is marked 17 and 18 in Fig. 1.

A preferred embodiment of the container 1 is shown in greater detail in Fig. 2—5. This container is provided with an inlet 2 and an outlet 4 and comprises the pressure-transmitting wall part 12 described above. The embodiment shown is intended for measurement of the pressure in blood. The upper wall 19 of the container has been designed therefore to be sunk or indented into the actual container whose inner space 20 is in communication with the inlet 2 via a duct 2a and with the outlet 4 via a duct 4a. In this manner the so-called "priming"-volume can be kept relatively low.

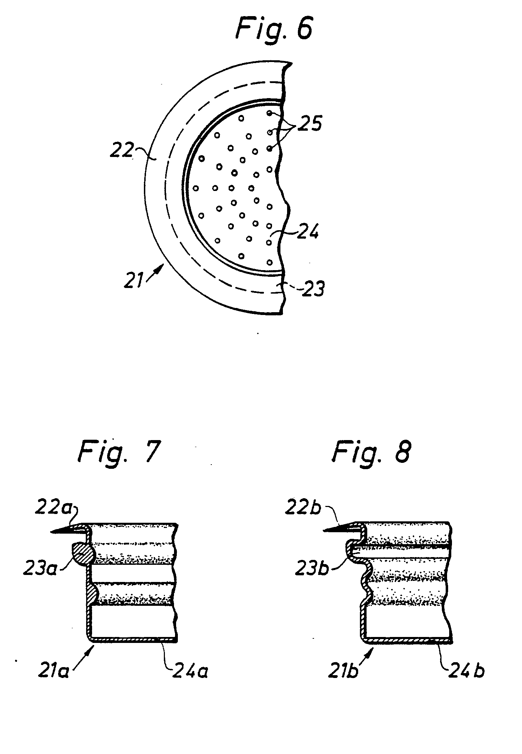

The sealing rings 13 and 14 shown in Fig. 1 can be replaced, if so desired, by the protective sleeve shown in Fig. 6 which in its entirety has been designated 21. In Fig. 7 and 8, which show two alternative embodiments of this protective sleeve, it has been designated instead 21a and 21b. As can be seen, the protective sleeve is provided with a lip seal 22 (22a or 22b respectively) which is intended to rest forming a seal against the machine front 7. It is provided moreover with an annular flange 23 (23a or 23b respectively) which together with the said lip flange is intended to replace the sealing rings 13 and 14. Finally, the bottom part 24 (24a or 24b respectively) is provided with a perforation 25. Thanks to this perforation this pressure-transmitting wall part 12 of the container 1 can readily fasten on by suction to the pressure-sensitive element 8 independently of the protective sleeve arranged in between.

Alternative embodiments

Naturally the invention is not limited solely to the particulars described above, but can be varied within the scope of the following claims. For example the container shown in Figures 2—5 is considered to be formed by blow moulding. Alternatively it may be produced, for example, by injection moulding, the shape then having to be adapted to this process. It may be appropriate in that case not to mould the pressure-transmitting wall part 12 in one piece with the remainder of the container 1. Instead it may be formed, for example, as a diaphragm clamped or welded to the container. The essential part is only that it should be so elastic or yielding that it does not substantially affect the measurement of the absolute pressure in the container 1. Moreover, of course, other components described in the system shown above may also be varied within wide limits.

**Claims**

1. A system for measuring the pressure of a medium comprising a pressure-sensitive element (8) and a container (1) provided with at least one inlet (2) the container being intended to communicate through this inlet (2) with the medium whose pressure is to be measured, characterized in that a pressure-transmitting wall part (12) of the container (1) is adapted so that with the help of a negative pressure it is fastened on by suction to the pressure-sensitive element (8) for measuring substantially the absolute pressure in the container.

2. A pressure measuring system in accordance with claim 1, characterized in that the pressure-sensitive element (8) is arranged sunk in a cavity (5) into which can be inserted at least a part of the said container (1) for contact between the pressure-sensitive element (8) and the pressure-transmitting wall part (12).

3. A pressure measuring system in accordance with claim 1, characterized in that the said cavity (5) is provided with sealing elements (13, 14) which are adapted so as to separate in a sealing manner the pressure-sensitive body (8) and the pressure-transmitting wall part (12) from the outside atmosphere when the container (1) is in inserted position in the cavity (5).

4. A pressure measuring system in accordance with claim 1, characterized in that the said container (1) is also provided with an outlet (4) and that it is intended that the medium whose pressure is to be measured should be able to flow through it.

5. A pressure measuring system in accordance with anyone of the preceding claims, characterized in that the part of the container (1) insertable in the cavity (5) is of substantially the same shape as the cavity itself.

6. A pressure measuring system in accordance with anyone of the preceding claims, characterized in that the part of the container (1) insertable in the cavity (5) is formed double-walled, and that the space (20) containing the medium between these walls (12, 19) is considerably smaller than the cavity (5) itself.

7. A pressure measuring system in accordance with anyone of the preceding claims, characterized in that the cavity (5) as well as the part of the container (1) insertable therein are substantially cylindrical and are sealed off against one another by means of one or more annular seals, e.g. O-rings (13, 14).

8. A pressure measuring system in accordance with anyone of the preceding claims, characterized by a thin and elastic protective sleeve (21) of substantially the same shape as the cavity (5) arranged between the cavity (5) and the part of the container insertable therein.

9. A pressure measuring system in accordance with claim 8, characterized in that the protective sleeve (21) is provided with sealing elements (23) to bring about a seal between the cavity (5) and the part of the container (1) insertable therein.

10. A pressure measuring system in accordance with claim 9, characterized in that the said sealing elements are in the form of one or more sealing rings (23a, 23b) moulded integrally with the sleeve.

11. A pressure measuring system in accordance with claim 8, characterized in that the inner part (24) of the protective sleeve (21) situated in inserted position closest to the pressure-sensitive element (8) is perforated so as to make possible a tight fastening on by suction of the pressure-transmitting wall part (12) of the container (1) to the pressure-sensitive element (8) essentially independently of the perforated part of the protective sleeve (21) situated in between.

## Patentansprüche

1. System zum Messen des Druckes eines Mediums mit einem druckempfindlichen Element (8) und einem Behälter (1), der mit mindestens einem Einlaß (2) versehen ist, wobei der Behälter über diesen Einlaß (2) mit dem Medium, dessen Druck zu messen ist, in Verbindung stehen soll, dadurch gekennzeichnet, daß ein Druckübermittlungswandteil (12) des Behälters (1) derart geeignet ausgestaltet ist, daß es mit Hilfe eines negativen Druckes durch Saugen am druckempfindlichen Element (8) zum Messen des im wesentlichen absoluten Druckes im Behälter befestigt ist.

2. Druckmeßsystem nach Anspruch 1, dadurch gekennzeichnet, daß das druckempfindliche Element (8) in einem Hohlraum (5) eingesenkt angeordnet ist, in welchen mindestens ein Teil des Behälters (1) für die Berührung zwischen dem druckempfindlichen Element (8) und dem Druckübermittlungswandteil (12) eingeführt werden kann.

3. Druckmeßsystem nach Anspruch 1, dadurch gekennzeichnet daß der Hohlraum (5) mit Abdichtelementen (13, 14) versehen ist, die geeignet derart ausgestaltet sind, daß sie in dichtender Weise den druckempfindlichen Körper (8) und das Druckübermittlungswandteil (12) gegen die Außenatmosphäre separieren, wenn der Behälter (1) sich in der im Hohlraum (5) eingefügten Stellung befindet.

4. Druckmeßsystem nach Anspruch 1, dadurch gekennzeichnet, daß der Behälter (1) auch mit einem Auslaß (4) versehen ist und daß es beabsichtigt ist, daß das Medium, dessen Druck gemessen werden soll, in der Lage sein sollte, durch ihn hindurchzuströmen.

5. Druckmeßsystem nach einem der vorhergehenden Ansprüche, dadurch gekennzeichnet, daß der Teil des Behälters (1), welcher in den Hohlraum (5) einsetzbar ist, im wesentlichen die gleiche Gestalt wie der Hohlraum selbst hat.

6. Druckmeßsystem dnach einem der vorhergehenden Ansprüche, dadurch gekennzeichnet, daß der in den Hohlraum (5) einsetzbare Teil des Behälters (1) doppelwandig ausgebildet ist und daß der Raum (20), welcher das Medium zwischen diesen Wänden (12, 19) enthält, erheblich kleiner als der Hohlraum (5) selbst ist.

7. Druckmeßsystem nach einem der vorhergehenden Ansprüche, dadurch gekennzeichnet, daß der Hohlraum (5) sowie der Teil des Behälters (1), der in diesen einsetzbar ist, im wesentlichen zylindrisch sind und mittels eines oder mehrerer Ringdichtungen, z.B. O-Ringe (13, 14), gegeneinander abgedichtet sind.

8. Druckmeßsystem nach einem der vorhergehenden Ansprüche, gekennzeichnet durch eine dünne une elastische Schutzhülse (21) von im wesentlichen derselben Gestalt wie der Hohlraum (5), die zwischen dem Hohlraum (5) und dem Teil des Behälters, der in diesen einsetzbar ist, angeordnet ist.

9. Druckmeßsystem nach Anspruch 8, dadurch gekennzeichnet, daß die Schutzhülse (21) mit Dichtelementen (23) versehen ist, um eine Dichtung zwischen dem Hohlraum (5) und dem Teil des Behälters (1) zu schaffen, der in diesen einsetzbar ist.

10. Druckmeßsystem nach Anspruch 9, dadurch gekennzeichnet, daß die Dichtelemente die Form eines oder mehrerer Dichtringe (23a, 23b) haben, die einstückig mit der Hülse geformt sind.

11. Druckmeßsystem nach Anspruch 8, dadurch gekennzeichnet, daß das innere Teil (24) der Schutzhülse (21), welches in eingesetzter Position dem druckempfindlichen Element (8) nächstliegend angeordnet ist, perforiert ist, um ein strammes Befestigen des Druckübermittlungswandteiles (12) des Behälters (1) an dem druckempfindlichen Element (8) durch Saugen zu ermöglichen, und zwar im wesentlichen unabhängig von dem perforierten Teil der dazwischen angeordneten Schutzhülse (21).

## Revendications

1. Système de mesure de la pression d'un fluide, comprenant un élément sensible à la pression (8) et un récipient ou réservoir (1) pourvu d'au moins une entrée (2), le réservoir étant prévu pour communiquer par cette entrée (2) avec le fluide dont la pression doit être mesurée, caractérisé en ce qu'une partie de paroi de transmission de pression (12) du réservoir (1) est prévue de sorte que, au moyen d'une pression négative, elle est fixée par succion à l'élément sensible à la pression (8) pour mesurer sensiblement la pression absolue dans le réservoir.

2. Système de mesure de pression suivant la revendication 1, caractérisé en ce que l'élément sensible à la pression (8) est encastré dans une cavité (5) dans laquelle on peut insérer au moins une partie dudit réservoir (1) pour établir le contact entre l'élément sensible à la pression (8) et la partie de paroi de transmission de pression (12).

3. Système de mesure de pression suivant la revendication 1, caractérisé en ce que ladite cavité (5) comporte des éléments d'étanchéité (13, 14) qui sont agencés de manière à séparer, de façon étanche, le corps sensible à la pression (8) et la partie de paroi de transmission de pression (12) de l'atmosphère extérieure lorsque le réservoir (1) est en position insérée dans la cavité (5).

4. Système de mesure de pression suivant la revendication 1, caractérisé en ce que ledit réservoir (1) comporte également une sortie (4) et en ce qu'il est prévu que le fluide dont la pression doit être mesurée puisse circuler à travers le réservoir.

5. Système de mesure de pression suivant l'une quelconque des revendications précédentes, caractérisé en ce que la partie du réservoir (1) qui peut être insérée dans la cavité (5) a sensiblement la même configuration que la cavité elle-même.

6. Système de mesure de pression suivant l'une quelconque des revendications précédentes, caractérisé en ce que la partie du réservoir (1) qui peut être insérée dans la cavité (5) est à double paroi et en ce que l'espace (20) contenant le fluide entre ces parois (12, 19) est beaucoup plus petit que la cavité (5) elle-même.

7. Système de mesure de pression suivant l'une quelconque des revendications précédentes, caractérisé en ce que la cavité (5) et la partie du réservoir (1) qui peut y être insérée sont sensiblement cylindriques et sont en contact étanche mutuel par l'intermédiaire d'une ou plusieurs garnitures annulaires d'étanchéité, par exemple des joints toriques (13, 14).

8. Système de mesure de pression suivant l'une quelconque des revendications précédentes, caractérisé en ce qu'il comprend un manchon protecteur mince et élastique (21) ayant sensiblement la même forme que la cavité (5) et placé entre la cavité (5) et la partie du réservoir qui peut y être insérée.

9. Système de mesure de pression suivant la revendication 8, caractérisé en ce que la manchon protecteur (21) comporte des éléments d'étanchéité (23) pour créer une étanchéité entre la cavité (5) et la partie du réservoir (1) qui peut y être insérée.

10. Système de mesure de pression suivant la revendication 9, caractérisé en ce que lesdits éléments d'étanchéité sont sous la forme d'un ou plusieurs anneaux d'étanchéité (23a, 23b) moulés solidairement avec le manchon.

11. Système de mesure de pression suivant la revendication 8, caractérisé en ce que la partie intérieure (24) du manchon protecteur (21), située en position insérée au plus près de l'élément sensible à la pression (8), est perforée de manière à permettre une fixation serrée, par succion, de la partie de paroi de transmission de pression (12) du réservoir (1) à l'élément sensible à la pression (8), sensiblement indépendamment de la partie perforée du manchon protecteur (21) située entre ces composants.

# Fig. 1

*Fig. 2*

*Fig. 3*

2

*Fig. 4*

*Fig. 5*

## Fig. 6

22

25

24

21

23

## Fig. 7

22a

23a

21a

24a

## Fig. 8

22b

23b

21b

24b